# EUROPEAN PATENT APPLICATION

(11) **EP 4 606 219 A1**
(43) Date of publication of application: **27.08.2025**
(21) Application number: 23810278.4
(22) Date of filing: 17.10.2023
(51) Int. Cl.: A01N 63/20, A01P 15/00, A01P 21/00, C12N 1/20

(54) **FLOWERING-INDUCING COMPOSITION**

(30) Priority: 18.10.2022 CU 20220062
(71) Applicant: Centro de Ingeniería Genética y Biotecnología, La Habana 11300 (CU)
(72) Inventor: MENA CAMPOS, Jesús, La Habana 17100 (CU); LEÓN BARRERAS, Licette de Jesús, La Habana 10600 (CU); MOREIRA RUBIO, Alain, Camaguey 70100 (CU); GARCIA SANTOS, Leyenis, La Habana 12500 (CU); GONZALEZ BLANCO, Sonia, La Habana 12100 (CU); WONG PADILLA, Idania, Camaguey 71300 (CU); PÉREZ GONZÁLEZ, Eikel, Camaguey 70100 (CU); RAMÍREZ NÚÑEZ, Yamilka, Camaguey 70100 (CU); BASULTO BAKER, Roberto, Camaguey 70300 (CU); GARCÍA SIVERIO, Marianela, La Habana 12100 (CU)
(74) Representative: V.O.
(86) International application number: PCT/CU2023/050004
(87) International publication number: WO 2024/083273

(57) **Abstract**

Flowering-inducing composition containing bacteria from Brevibacterium celere C-924 strain and excipients or stabilizers. The mentioned agent can increase the number of flowers and produce an earlier inflorescence in cultivated plants. A method to induce plant flowering based on administering a composition containing bacteria from Brevibacterium celere C-924 strain to the soil or a substrate. Utilization of bacteria from Brevibacterium celere C-924 strain to produce a flowering-inducing composition in plants.

## Description

### Field of technology

This invention relates to soil microbiology, agriculture, and the application of non-hormonal flowering inducers. Specifically, this invention reveals a flowering-inducing composition capable of replacing hormonal or chemical flowering inducers without affecting the biological balance or the environment.

### Prior state-of-the-art

There is a growing interest in using native and non-native beneficial microorganisms to improve crop yields and raise food production. It has been associated with increased problems linked to the use of chemicals (including hormones) in agriculture and their harmful effects on human health and the environment.

The soil contains several microorganisms with that potential, which have been introduced for various integrated pest practices and increased crop productivity (Avis et al. Soil Biology and Biochemistry 40(7): 1773-1740, 2008). Several growth regulators can enhance crop yields, and an additional number of them are being studied for application at different stages of plant development and productivity to stabilize production.

The roots have a biological and physical effect on the rhizosphere, the root-surrounding soil. Rhizobacteria constitute a group of bacteria with phylogenetic differences, which compose the rhizosphere and force out other microorganisms from this nutrient-rich region. Besides, these bacteria may interact with plants that promote or inhibit their growth (Kloepper et al. Curr.Microbiol.4: 317-320, 1980).

Any particular microorganism may affect plants through several mechanisms, while the cultivating conditions may invalidate or accentuate the effects of interactions (Carrillo-Castañeda et al. Biotecnologia Aplicada. 17: 171-176, 2000). Major case studies have provided evidence that volatile organic compounds released by various microorganisms, such as *Bacillus* sp., *Pseudomonas* sp., *Arthrobacter* sp., *Fusarium* sp., and *Alternaria* sp. could stimulate growth in specific "target" plantlets, like *Arabidopsis* and tobacco (Fincheira and Quiroy. Microbiological Research, pp 63-75,2018).

Growth stimulation due to the effect of the spermosphere and rhizosphere involves saprotrophs, though different interactions may cause selective increments of specific symbiont groups (Whipps. Journal of Experimental Botany 2001; Vol. 52, No. 90001, pp. 487-511).

Certain rhizosphere bacteria may stimulate plant growth and limit the attacks of soil and seed pathogenic agents (Zablotowicz, R. M. et al. The rhizosphere and plant growth. D.L. Keister and P.B. Cregan (ed.), Kluwer Academic Publishers, Dordrecht. The Netherlands, 1991, p. 315-326). Bacterial strain C-924, revealed in patent EP0774906, is a practical example of the above. Initially, the strain was evaluated and classified as *Corynebacterium paurometabolum,* using biochemical methods, API-50 CH reference. The strain was deposited by the Center of Genetic Engineering and Biotechnology (CIGB) of Cuba, in Centraalbureau Voor Schimmelcultures, Baarn, The Netherlands (Deposit No. CBS 613.95). Several reports associated it with broad pesticide and antiparasitic activities. Species *Corynebacterium paurometabolum* was later classified as *Tsukamurella paurometabola.* The biofertilizer activity of this antibacterial strain, called C-924 (Patent EP2154121) was further demonstrated. Then it was reassessed through molecular, biochemical, morphological, and physiological methods at the Institute of Microbiology, Chinese Academy of Science, where it was identified as *Brevibacterium celere.*

Flower induction is a physiological process in which the metabolism of hormones undergoes a qualitative change. Previous flowering initiation, according to Ramirez et al. (Hort Science 45(10: 1453-1458, 2010)), is defined as the cell division and elongation that originates the early flower shoots, which might be reproductive (just flowers), vegetative (just the leaves), or mixed (leaves and flowers in the same inflorescence). Flower induction in fruit trees is a process in which the buds, initially vegetative, undergo metabolic changes to turn into flower buds. Environmental, ontogenetic, and physiological factors can control this process. Rev. Plant Physiol. 39: 175-219, 1988).

The knowledge of flower induction physiology permits harvest programming using chemicals, biologicals, and other crop-handling techniques that help prolong or delay the high fruit season. Some particular crops can be produced outside their natural adaptation zone (forcing production), increasing competitiveness in the national and international markets and encouraging efficient use of inputs, such as fertilizers and pesticides (Davenport and Nuñez. CAB International. 1997, pp 69-146). Several regulating ways of controlling flowering and flower development today are varied and complex, and demand strict control of gene expression and protein levels. (Hong and Jackson. Plant Biotechnology Journal, pp 1-11, 2015).

Classic plant hormones are the first factors thought of as the chemical components that influence flowering time. However, designing a common scheme for the metabolic network that regulates plant hormones in different plant species linked to flowering time is difficult. Surface application of plant hormones might potentially hinder several processes (Ionescu et al. Journal of Experimental Botany, 68, 3, 2016). Moreover, multiple examples have shown that plant hormones may cause adverse effects on human health. (https://www.conasi.eu/blog/consejos-de-salud/fitoestrogenos).

Some chemicals can increase the formation of floral primordia considerably when induction is desired. For instance, to increase yields in papaya (*Carica papaya* L.) Benzotiazol^{™} has been effective, with 66.7% production increments (Engormix, https://www.engormix.com, 2020); however, these hormonal treatments are refused by ecologists and the average consumers.

Accordingly, there is a need to identify new environmentally friendly flowering inducers with no adverse effects on consumer health.

### DETAILED DESCRIPTION OF THE INVENTION

This invention addresses the above-mentioned problem, with a flower-inducing composition that contains *B. celere* C-924 microbial strain bacteria and excipients or stabilizers. The mentioned strain was deposited under the number CBS 613.95. In one embodiment of this invention, the bacterial composition is between 10⁶ cfu and 10¹² cfu per gram of solid composition or mL of liquid composition.

Availability of a *B. celere* microorganism in the soil (Ivanova et al. Int. J. Syst. Evol. Microbiol. 54(Pt 6): 2107-2111, 2004), which can act as a flowering inducer, minimize or prevent the utilization of other inducers. Most hormones and chemical products cause adverse effects on the environment and human health. However, the current invention addresses the complex issue of flowering inducer utilization in various crops that need it.

This bacterial strain was isolated from the rhizosphere and applied to the soil rather than the foliage; hence, it is environmentally friendly and has no harmful effects on human health. Certainty, a microbial flowering inducer such as the *B. celere* C-924 strain could address the technological issue, which constitutes a novelty in this area of technology. So far, there are no examples of microorganisms used as flower inducers. In an implementation of this invention, the capacity of strain C-924 to have a positive effect on the flowers of several cultivated plants was demonstrated.

The application of *the B. celere* C-924 bacterial strain to the soil is effective on an organic substrate (chemically active), in combination with an organic matter or amino acid carrier, or both. The microorganism is applied as an aqueous cellular solution. In one embodiment, it is applied as a powder, at a concentration of 10¹² cfu/g solid composition, in 5 - 25 g 10 L of water before application to the soil or the substrate.

This invention consists of a method to induce plant flowering based on the administration of a composition containing bacteria from *the B celere* C-924 strain to the soil or substrate. In one embodiment of this invention, in the mentioned method, the bacterial concentration in the composition is between 10⁶ cfu and 10¹² cfu per gram of solid or liquid composition (mL).

Solanaceous plants (tomatoes), cucurbitaceous (cucumbers), musaceous (bananas), myrtaceous (guavas), Euphorbiaceae (Sacha Inchi), and ornamental plants (roses) are some examples of economically significant plants where microbial C-924 strain could be used effectively.

In one particular embodiment of the invention method, plants are selected from a group made up of tomatoes (*Lycopersicon esculentum* Mill), cucumbers (*Cucumis sativus*)*,* bananas (*Musa* sp.), guavas (*Psidium guajava* L.), and Sacha Inchi (*Plukenetia volubilis* L.). In another embodiment, the plants are ornamental. In a particular embodiment of the invention, the ornamental plant is the rose (*Rosa* sp).

In the invention method, the administration of the composition applied to the soil or substrate is made before, during, or after sowing. In such a method, the composition of C-924 is administered in combination or mixed with other microorganisms. Another objective of the invention is the utilization of bacteria from the *B celere* C-924 strain to produce a flower-inducing composition in plants.

### Examples of embodiment

### Example 1. Effect of Brevibacterium celere C-924 bacterial strain on tomato flowering.

The influence of *Brevibacterium celere* C-924 bacterial strain on tomato (*Lycopersicon esculentum* Mill, hybrid FA-180) flowering was studied. It was done in a 0.09 ha protected cultivation greenhouse, with sialitic brown soil (Hernández-Jiménez et al. Cultrop, 2019; vol. 40 no. 1: a15-e15), with 3% labile organic matter and 12% total organic matter. The soil was free from parasitic plant nematodes and pathogenic microorganisms. The cultivation house was divided into 16 plots (19.25 m² each), using an experimental design with four treatments and four replications, as follows:
**1. A treatment with a concentrated aqueous suspension (5.0 x 10¹¹ cfu/mL) of C-924 bacteria,** 10 L/ha was applied through the ferti-irrigation system before transplanting the tomato plantlets. The final suspension applied in the plant rhizosphere was 5.0 x 10⁷ cfu/mL of the suspension. Other two applications were made every 21 days during the crop cycle. The other actions in this treatment coincided with the **control treatment (No. 4).**
**2. A treatment with a concentrated aqueous suspension (5.0 x 10¹¹ cfu/mL) of C-924 strain bacteria** at 4 L/ha was applied through the ferti-irrigation system before transplantation. The final suspension applied in the plant rhizosphere was 5.0 x 10⁶ cfu/mL of the suspension. Other two applications were made every 21 days during the crop cycle. The other actions in this treatment coincided with the **control treatment (No. 4).**
**3. Hormonal treatment: Hormoton^{™} 2SL** (5 mL/L water), through foliar spraying (twice a week), according to the Manual para la Produccion Protegida de Hortalizas (Casanova et al. https://isbn.cloud/9789803183271/manual-para-la-produccion-protegida-de-hortalizas-version-digital, 2007). The hormone has a positive effect on flower pollen production and fertility. The other actions in this treatment coincided with the **control treatment (No. 4).**
**4. Control treatment: Without flowering inducers.** The crop was handled according to the instructions in the *Manual para la* Producción Protegida de Hortalizas (Casanova et al., 2007), except for the application of flowering inducers, since none was used in this treatment.

The treatments with the bacterial suspension were performed to the localized irrigation system (drip) using an additional pump, following total irrigation of 1 L per square meter, and according to the instructions given in the Manual para la Produccion Protegida de Hortalizas (Casanova et al., 2007).

The percentage of flowering plants was determined 15 days following transplantation. The following plant parameters were measured and quantified (40 plants) every 15 days:
- Percent of flowering plants.
- Number of flower clusters per plant.
- Number of flowers per plant.

Table 1 summarizes the results of the first evaluation 15 days after the transplantation. The percentage (%) of flowering plants, the number of flower clusters per plant, and the number of flowers per plant were significantly higher in the treatments that received *B. celere* C-924 bacterial strain in any of the doses. No statistically significant differences were observed between the results of the variables measured for the two concentrations of the *B. celere* bacterial strain (treatments 1 and 2), nor between the results without the microorganism. Additionally, the floral primordia emerged a week earlier in treatments 1 and 2 than in treatments 3 and 4. The study demonstrated the properties of this microorganism as a flower inducer in field conditions.

**Table 1. Means of parameters evaluated in every treatment.**

| **Treatment** | **Flowering plant %** | **Cluster/plant** | **Flowers/plant** |
|---|---|---|---|
| 1 | 99.4%^{a} | 1.58^{a} | 9.13^{a} |
| 2 | 93.1%^{a} | 1.49^{a} | 8.98^{a} |
| 3 | 75.6 %^{b} | 1.26^{b} | 7.99^{b} |
| 4 (Control) | 67.5 %^{b} | 1.20^{b} | 7.73 ^{b} |

| | | | |
|---|---|---|---|
| Means with different scripts are significantly different, according to Tukey, (p ≤ 0.05). The parameters were evaluated 15 days after transplantation. | | | |

Table 2 shows the number of clusters per plant and flowers per plant 60 days after transplantation.

**Table 2. Means of parameters evaluated in every treatment.**

| **Treatment** | **Cluster/plant** | **Flowers and fruit/plant** |
|---|---|---|
| 1 | 7.09^{a} | 26.35^{a} |
| 2 | 6.89^{ab} | 25.09^{a} |
| 3 | 6.82^{b} | 25.01^{a} |
| 4 (Control) | 5.32^{c} | 19.64^{b} |

| | | |
|---|---|---|
| Means with different scripts are significantly different, according to Tukey (p ≤ 0.05). The parameters were evaluated 60 days after transplantation. | | |

The parameter values observed in treatments 1, 2, and 3 were significantly higher than in treatment 4 (control with no flowering inducer). Surprisingly, no statistically significant differences were observed in treatments 1, 2, and 3. Treatment 4 showed high levels of flower abortion and no fruit formation during the crop cycle.

Consequently, using C-924 as a flowering inducer in tomato plants was favorable, with increases in the number of flowers and fruits. Moreover, the microbial inducer may replace the hormonal treatment refused by ecologists and consumers.

### Example 2. Effect of Brevibacterium celere C-924 bacterial strain on cucumber flowering

The influence of *B celere* C-924 bacterial strain on cucumber (*Cucumis sativus,* var. Tropical SS-5) flowering was studied under semi-protected organoponic conditions with a chemically active substrate. A row containing a chemically active organic substrate, and over 200 plants (free from parasitic nematodes and plant pathogenic microorganisms) in 25% red ferralitic soil (Hernández-Jiménez et al. Cultrop, 2019; vol. 40 no. 1: a15-e15), 25% zeolite, and 50% worm humus was selected (Rodriguez et al. Manual Técnico para Organopónicos, Huertos Intensivos y Organoponía Semiprotegida. Sexta Edición, ISBN: 959-246-030-2, 2007).

The row was split into 9 plots with 20 plants each, used as experimental units. The experimental design consisted of three treatments and three replications, and was performed as follows:
**1. A treatment with a suspension consisting of a powder formulation (2.0 x 10¹² cfu/g) of C-924 bacterial strain.** The suspension consisted of a 2.5 kg dose of the powder/ha. The previously prepared aqueous suspension was applied using a watering can before sow the cucumber seeds directly. The final suspension applied in the plant rhizosphere had a 10⁷ cfu/mL concentration. Other two applications were made every 21 days, during the crop cycle. The other actions in this treatment coincided with the control treatment 3 (control).
**2. A treatment with a powder formulation (2.0 x 10¹² cfu/g) of C-924 bacterial strain.** The suspension required 1 kg of powder/ha. The previously prepared aqueous suspension was applied using a watering can before sow the cucumber seeds directly. The final suspension applied in the plant rhizosphere had a 10⁶ cfu/mL concentration. Two other applications were made during the crop cycle every 21 days. The other actions in this treatment coincided with the control treatment 3 (control).
**3. Control: Without flowering inducers.** The crop was handled according to the Manual Técnico para Organopónicos, Huertos Intensivos y Organoponía Semiprotegida. (Rodriguez et al. Sexta Edición, ISBN: 959-246-030-2, 2007).

The two *B. celere* C-924 bacterial strain treatments were irrigated with a watering can; supplying 0.25 L of suspension per plant.

From the beginning of flowering through the first harvest (30-45 days after sowing), the flowers per plant in each treatment were quantified. The outcomes are shown in Table 3.

**Table 3. Number of flowers mean per plant from the beginning of flowering to the first harvest.**

| **Treatments** | **Female Flowers/plant** | **Male flowers/plant** | **Total flowers/plant** |
|---|---|---|---|
| 1 | 3.05^{a} | 10.15^{a} | 13.20^{a} |
| 2 | 2.80^{a} | 9.78^{a} | 12.58^{a} |
| 3 Control | 1.92^{b} | 8.97^{b} | 10.89^{b} |

| | | | |
|---|---|---|---|
| Means with equal scripts do not differ significantly, according to Tukey's multiple ranks sum test (p ≤ 0.05). | | | |

The number of flowers was higher in the C-924-treated plants. The results evidenced a statistically significant influence of the strain to induce early cucumber flowering. Therefore, its use as a flowering inducer in cucumber is recommended.

### Example 3. Effect of Brevibacterium celere C-924 bacterial strain on banana flowering.

A study was conducted to determine the effect of C-924 on banana flowering, var. Cavendish *"Grande Naine"* (Big Dwarf), *Musa* sp. hybrid. A plot containing dark red ferritic soil free from plant parasitic nematodes and plant pathogenic microorganisms was selected. Cultrop, 2019; vol. 40 no. 1: a15-e15). The soil was previously fertilized, and various organic improvements were performed (Instructivo Técnico del Cultivo del Plátano: Ministerio de la Agricultura, Cuba, 2012).

The experimental field consisted of a Latin square design with four treatments and four replications. The plots were marked on double rows of 20 plants each (future saplings), accounting for 100 m² each. Irrigation was automatic, using localized micro jets.

The following treatments were applied:
**1. A treatment with an aqueous suspension containing 10⁷ cfu of** recently fermented biomass obtained directly from the microorganisms in the bacterial strain C-924/mL water. The bacterial suspension was administered using a watering can when transplanting the banana plantlets from the nursery. The volume of the final suspension applied in the plant rhizosphere was 10 L per plant.
   Other three applications were made every 2 months, which coincided with the vegetative-reproductive phase described for this cultivar, according to Martinez and Cayón (Rev. Fac. Nac. Agr. Medellin 64:6055-6064, 2011). The other actions in this treatment coincided with treatment 3 (control).
**2. A treatment with a suspension containing 10⁶ cfu of** recently fermented biomass from the microorganisms in bacterial strain C-924/mL water. The bacterial suspension was administered using a watering can at the moment of transplanting the banana plantlets from the nursery. The volume of the final suspension applied in the plant rhizosphere was 10 L per plant. Other three applications were made every 2 months, which coincided with the vegetative-reproductive phase described for this cultivar, according to Martinez and Cayón (2011). The other actions in this treatment coincided with treatment 3 (control).
**3. Control: Without flowering inducers.** The crop was handled according to the Instructivo técnico del cultivo del plátano (Instituto de Investigaciones de Viandas Tropicales, 2012).

Each of the 20 plants in the plots was measured and quantified between days 116 and 235 following the transplantation of the plantlets from the nursery. This period included the vegetative-reproductive and reproductive-productive phases of this banana cultivar (Martinez and Cayón, 2011), the time calculated for flowering (173 days after transplanting), and until the end of harvest (235 days).

The parameter observed was the number of days of early flowering (plant average) in treatments 1 and 2, compared to the control (treatment 3). The results are shown in Table 4.

**Table 4 Means of early flowering per plant**

| **Treatments** | **Daily means of early flowering per plant** |
|---|---|
| 1 | 8.15 ^{a} (earlier than the control). |
| 2 | 5.85 ^{a} (earlier than the control). |
| 3 Control | 0.00 ^{c} |

| | |
|---|---|
| Means with equal scripts do not differ significantly, according to Tukey's multiple ranks test (p≤0.05). | |

The application of C-924 at both concentrations caused early flowering in banana plants. Accordingly, C-924 is recommended as a flowering inducer in banana.

### Example 4. Effect of Brevibacterium celere C-924 bacterial strain on guava flowering.

A study was conducted to determine the flowering effect of C-924 on guava (*Psidium guajava* L.), cultivar E.E.A 18-40 (Enana Roja Cubana). A guava field in brown sialitic soil, free from parasitic nematodes and plant pathogenic microorganisms was selected (Hernández-Jiménez et al. Cultrop, 2019; vol. 40 no. 1: a15-e15).

The plots were marked on single rows of 4 blocks containing 80 plants each; every block accounted for 800 m². The other actions to guava were performed according to the Instructivo técnico para el cultivo de la guayaba. Various authors. Instituto de Investigaciones en Fruticultura Tropical. Ministerio de la Agricultura, Cuba, 2012). Asociación Cubana de Técnicos Agricolas y Forestales. Cuba, Primera edición: 2011.

The following treatments were applied accordingly:
**1. A treatment with a suspension consisting of a C-924 strain powder formulation (2.0 x 10¹² cfu/g of the formulation).** A 2 kg dose of the powder/ha was used to prepare the suspension. The previously prepared aqueous suspension was applied using a watering can. It was immediately followed by leaf trimming that included all the flower shoots observed in the 4 experimental blocks. The final suspension applied in the plant rhizosphere had a 1.25 x 10⁷ cfu/mL concentration. Every plant received approximately 2 g of the formulation resuspended in 5 L. The other actions in this treatment coincided with treatment 4 (control).
**2. A treatment with a suspension consisting of a C-924 strain powder formulation (2.0 x 10¹² cfu/g of the formulation).** The suspension consisted of a 2.5 kg dose of the powder/ha. The previously prepared aqueous suspension was applied using a watering can. It was immediately followed by leaf trimming that included all the flower shoots observed in the four experimental blocks. The final suspension applied in the plant rhizosphere had a 1.25 x 10⁷ cfu/mL concentration. Every plant received approximately 2.5 g of the formulation resuspended in 5 L. The other actions in this treatment coincided with treatment 4 (control).
**3. A treatment with a suspension consisting of a C-924 strain powder formulation (2.0 x 10¹² cfu/g of the formulation).** A 3 g dose of the powder/ha was used to prepare the suspension. The previously prepared aqueous suspension was applied using a watering can. It was immediately followed by leaf trimming that included all the flower shoots observed in the four experimental blocks. The concentration of the final suspension applied to the plant rhizosphere was 1.5 x 10⁷ cfu/mL. Every plant received approximately 3 g of the formulation resuspended in 5 L. The other actions in this treatment coincided with treatment 4 (control).
**4. Control: Without flowering inducers.** The crop was handled according to the Instructivo técnico para el cultivo de la guayaba (Various authors Instituto de Investigaciones en Fruticultura Tropical. Ministerio de la Agricultura, Cuba, 2012). Instituto de Investigaciones en Fruticultura Tropical Asociación Cubana de Técnicos Agricolas y Forestales. Cuba, Primera edición: 2011).

Flower counts were performed in all 80 plants (each regarded as an experimental unit), in every plot (or block), between day 0 (trimming and application of the product) and day 45, considering the following parameters:
- Days until flowering shoots in the first 20 plants of each treatment.
- Flower shoot average observed per plant in each treatment until 45 days after trimming and treatments.

Table 5 shows the results of the study.

**Table 5. Means of the parameters evaluated.**

| **Treatments** | **Days until flowering shoots (days/plant)** | **Flower shoots/plant** |
|---|---|---|
| 1 | 9.8^{b} | 83.2^{a} |
| 2 | 8.7^{bc} | 89.0^{a} |
| 3 | 7.6^{c} | 89.4^{a} |
| 4 Control | 15.2^{a} | 41.9^{b} |

| | | |
|---|---|---|
| Means with equal scripts do not differ significantly, according to Tukey's multiple ranks sum test (p≤0.05). | | |

The results shown in Table 5 evidenced a significant effect of C-924 on guava flowering induction, both concerning early shooting and increased flower shoots. Hence, its use is recommended as a flowering inducer for this crop.

### Example 5. Effect of Brevibacterium celere C-924 bacterial strain on Sacha Inchi flowering.

A **Sacha Inchi (*Plukenetia volubilis* L.)** field in red ferralitic soil, free from parasitic nematodes and plant pathogenic microorganisms was selected (Hernández-Jiménez et al. Cultrop, 2019; vol. 40 no. 1: a15-e15). The plots were marked on single rows of 3 blocks each, containing 20 plants each; each block covered 180 m². The other actions were performed according to the Instructions Manual. Manual de capacitación. Cultivo de Sacha Inchi (Plukenetia volubilis L.)", 2008 (https://www.academia.edu/19770262/MANUAL_SACHA_INCHI).

The following treatments were applied:
**1. Treatment with a suspension consisting of a powder formulation (5.0 x 10¹¹ cfu/g) of C-924 bacterial strain in a 2.5 kg/ha dose,** applied using a watering can, in which the powder was resuspended in water. It was immediately followed by trimming that included all the flower shoots observed in the three experimental blocks. The final suspension applied in the plant rhizosphere was 10⁷ cfu/mL of the suspension. Each plant received approximately 2.25 g of the formulation resuspended in 5 L of water. The other actions in this treatment coincided with treatment 3 (control).
**2. Treatment with Gibberellic acid (60 mg/L)** applied with a foliar sprayer (Pezo et al. Scientia Agropecuaria 10(4): 455-460, 2019). The treatment was preceded by trimming that included the elimination of all the flower shoots observed in the 3 experimental blocks. The other actions in this treatment coincided with treatment 3 (control).
**3. Control: Without flowering inducers.** The crop was handled according to the Manual de capacitación. Cultivo de Sacha Inchi (Plukenetia volubilis L.)", 2008 (https://www.academia.edu/19770262/MANUAL_SACHA_INCHI). Flower counts were performed in all 20 plants (each regarded as an experimental unit), in every plot (or block), between day 0 (application of the product) and day 90, considering the following parameters:
   - Days until the first female flowers appeared in the 20 plants of each treatment.
   - Female flower shoot average observed per plant in each treatment until 90 days after the treatments.

The results from parameter evaluation and count are shown in Table 6.

**Table 6. Means of the parameters evaluated.**

| **Treatments** | **Days until the first female flowers appeared (days/plant)** | **Total female flowers/plant** |
|---|---|---|
| 1 | 19.0^{b} | 445.7^{a} |
| 2 | 18.1^{b} | 459.3^{a} |
| 3 Control | 29.4^{a} | 237.6^{b} |

| | | |
|---|---|---|
| Means with equal scripts do not differ significantly according to Tukey's multiple ranks sum test (p<0.05). | | |

These results evidenced a significant effect of C-924 on Sacha Inchi flowering induction, both concerning early shooting and an increased number of female flower shoots. No significant differences were observed with the utilization of Gibberellic acid, a well-known flowering inducer of several crops, including Sacha Inchi (Pezo et al. Scientia Agropecuaria 10(4): 455-460, 2019). In that case, the advantage of C-924 bacteria as flowering inducers, instead of the Gibberellic acid (hormone) lies in the substitution of a hormonal product by a microorganism from the rhizosphere.

### Example 6. Effect of Brevibacterium celere C-924 bacterial strain on rose flowering.

The effect of the C-924 strain on red rose (*Rosa* sp) flowering was studied in a chemically active organic substrate (free from parasitic nematodes and plant pathogenic microorganisms) made up of 25% red ferralitic soil (Hernández-Jiménez *et al*.)*,* 25 % zeolite and 50 % worm humus (Rodriguez et al., 2007).

The substrate was deposited in 60 pots with a 20 L capacity. Then the red rose (*Rosa* sp.) cuttings were planted, in greenhouse conditions (protected cultivations houses under plastic) until they became rose-producing plants (Yong. Cultivos Tropicales, vol. 25, no. 4, p. 53-60, 2004). The experimental design consisted of four treatments with 20 replications (each plant was considered an experimental unit), and treated as follows:
**1. A treatment using a solid formulation of C-924 (5.0 x 10¹¹ cfu/g of powder) in a 2.5 g/pot dose** applied in an aqueous suspension using a watering can, following trimming that eliminated all flower shoots. The final aqueous solution was applied to each of the 20 pots (plants), in a 1.25 x 10¹² cfu dose, and the bacteria were resuspended in 2 L of water. The other actions in this treatment coincided with treatment 4 (control).
**2. A treatment using a solid formulation of C-924 (5.0 x 10¹¹ cfu/g of powder) in a 1 g/pot dose,** was applied in an aqueous suspension using a watering can, following trimming that eliminated all flower shoots. The final aqueous solution was applied to each of the 20 pots (plants), in a 5 x 10¹¹ cfu dose, and the bacteria were resuspended in 2 L of water. The other actions in this treatment coincided with treatment 4 (control).
**3. A treatment using a commercial formulation of *Bacillus subtilis* (Fusvicur^{®}.** https://www.fertilizantesyabonos.com/fusvicur) **in a 5 g/pot dose,** applied in an aqueous suspension using a watering can, following trimming which eliminated all flower shoots. The final aqueous solution was applied to each of the 20 pots (plants). The other actions in this treatment coincided with treatment 4 (control).
**4. Control: Without flowering inducers.** As described in the three previous treatments, trimming was performed to eliminate all flower shoots. The crop was handled according to the instructions by Yong (Cultivos Tropicales, vol. 25, núm. 2, pp. 53-67 Instituto Nacional de Ciencias Agricolas La Habana, Cuba, 2004).

Flower counts were performed in all 20 plants (each regarded as an experimental unit), between day 0 (application of the product) and day 60, considering the following parameters:
- Days until the first flowers appeared in the 20 plants of each treatment.
- Flower shoot average observed per plant in each treatment, until 90 days following the treatments.

**Table 7. Means of the parameters evaluated.**

| **Treatments** | **Days until the first flowers appeared in 20 plants (days/plant)** | **Total flowers/plant** |
|---|---|---|
| 1 | 8.5^{c} | 156.6^{a} |
| 2 | 12.6^{b} | 141.1^{b} |
| 3 | 17.9^{a} | 129.0^{c} |
| 4 (Control) | 17.4^{a} | 124.8^{c} |

| | | |
|---|---|---|
| Means with equal scripts do not differ significantly according to Tukey's multiple ranks sum test (p≤0.05). | | |

The results shown in Table 7 evidenced a significant effect of C-924 on rose flowering induction, both concerning early shooting and increased flower shoots, thus showing its feasibility as a flowering inducer for ornamental plants. Moreover, the well-known biofertilizer and biostimulant containing *Bacillus subtilis* (Fusvicur^{®}), showed no positive effects as a flowering inducer under these conditions.

## Claims

1. Flowering-inducing composition comprising strain C-924 of the bacterium *Brevibacterium celere,* deposited on August 5, 1995 with Deposit No. CBS 613.95 at the *Centraalbureau voor Schimmelcultures (CBS), Baarn,* Netherlands, and excipients or stabilizers, at a concentration between 10⁶ and 10¹² colony forming units (cfu) per gram of solid composition or mL of liquid composition.

2. Method for inducing flowering in plants that comprises the administration to the soil or substrate of a composition comprising the C-924 strain of the *Brevibacterium celere* bacteria.

3. The method of claim 2 wherein the concentration of the bacteria in the composition is between 10⁶ cfu and 10¹² cfu per gram of solid composition or mL of liquid composition.

4. The method according to claim 2 wherein the plant is selected from the group consisting of tomato (*Lycopersicon esculentum* Mill), cucumber (*Cucumis sativus*)*,* banana (*Musa sp*.)*,* guava (*Psidium guajava L*.) and Sacha Inchi (*Plukenetia volubilis L*.)*.*

5. The method according to claim 2 wherein the plant is ornamental.

6. The method according to claim 5 wherein the ornamental plant is the rose (*Rosa sp*).

7. The method according to claim 2 wherein the administration of the composition is carried out before, during or after planting the plant.

8. Use of the C-924 strain of the bacteria *Brevibacterium celere* for the manufacture of a plant flowering-inducing composition.
